# EUROPÄISCHE PATENTSCHRIFT

(11) **EP 2 047 071 B1**
(45) Veröffentlichungstag und Bekanntmachung des Hinweises auf die Patenterteilung: **27.11.2013**
(21) Anmeldenummer: 07786251.4
(22) Anmeldetag: 21.07.2007
(51) Int. Cl.: C07C 29/151, C10G 2/00, F01K 17/04, F01K 13/00

(54) **VERFAHREN ZUR REDUZIERUNG DER CO2-EMISSION FOSSIL BEFEUERTER KRAFTWERKSANLAGEN**
METHOD FOR REDUCING THE CO2 EMISSION OF FOSSIL-FUELLED POWER GENERATING PLANTS
PROCÉDÉ POUR RÉDUIRE LES ÉMISSIONS DE CO2 DE CENTRALES À COMBUSTIBLES FOSSILES

(30) Priorität: 27.07.2006 DE 102006034712
(43) Veröffentlichungstag der Anmeldung: 15.04.2009
(73) Patentinhaber: STEAG New Energies GmbH, 66111 Saarbrücken (DE); IZES gGmbH, 66115 Saarbrücken (DE)
(72) Erfinder: DENGEL, Andreas, 66539 Neunkirchen (DE); DÖRR, Heinz-Kurt, 66386 St. Ingbert-Hassel (DE); GROSS, Bodo, 66292 Riegelsberg (DE)
(74) Vertreter: Bernhardt, Reinhold
(86) Internationale Anmeldenummer: PCT/EP2007/006505
(87) Internationale Veröffentlichungsnummer: WO 2008/012039

(56) Entgegenhaltungen:
- EP-A- 0 128 404
- EP-A- 0 413 199
- EP-A- 0 539 244
- US-A- 5 342 702
- NANDJEE ET M ROCHE F: "Nouveaux cycles de turbines à gaz au méthanol" REVUE GENERALE DE THERMIQUE, ELSEVIER EDITIONS SCIENTIFIQUES ET MEDICALES,PARIS, FR, Bd. 25, Nr. 289, Januar 1986 (1986-01), Seiten 19-26, XP002085579 ISSN: 0035-3159

## Beschreibung

Die Erfindung betrifft ein Verfahren zur Reduzierung der CO₂-Emission fossil befeuerter Kraftwerksanlagen, bei dem aus Wasserstoff und im Rauchgas der Kraftwerksanlage enthaltenen Kohlendioxid Kohlenwasserstoffe synthetisiert werden und bei dem der Wasserstoff elektrolytisch unter Einsatz eines Teils der durch Kraftwerksanlage erzeugten Elektroenergie gewonnen wird.

Verfahren solcher Art gehen aus der US-A-5342702 und der EP-A-0529244 hervor. Zur Synthese von Methanol verwandter Wasserstoff wird vorzugsweise in Phasen geringen Elektroenergiebedarfs, insbesondere während der Nachtstunden erzeugt und zwischengespeichert.

Der Erfindung liegt die Aufgabe zugrunde, ein neues Verfahren der eingangs erwähnten Art zu schaffen, das eine Reduzierung der CO2-Emission von Kraffwerken bei verringertem Energie- und Bauaufwand, insbesondere für die Wasserstoffgewinnung, ermöglicht.

Das diese Aufgabe lösende Verfahren nach der Erfindung ist dadurch gekennzeichnet, dass zur elektrolytischen Wasserstoffgewinnung eine im Rahmen der Primärregelung der Netzfrequenz kurzfristig abrufbare Reserve der durch die Kraffwerksanlage erzeugten Elektroenergie eingesetzt wird und dass für die Synthese ggf. erforderliche Wärme aus dem Rauchgas ausgekoppelt und bei der Synthese frei werdende Wärme in die Verbrennungsluft eingekoppelt wird.

Der benötigte Wasserstoff wird elektrolytisch unter Einsatz eines Teils der durch die Kraftwerksanlage selbst erzeugten Elektroenergie gewonnen werden, wobei eine im Rahmen der Primärregelung der Netzfrequenz kurzfristig abrufbare Reserve der durch die Kraftwerksanlage erzeugten Elektroenergie zur Wasserstoffgewinnung eingesetzt wird, wie dies in der deutschen Patentanmeldung 10 2005 001 009.1 beschrieben ist. Im Falle einer erforderlichen Stützung der Netzfrequenz im Rahmen der Primärregelung lässt sich die Elektrolyse unterbrechen und der betreffende Energieanteil kurzfristig ins Netz einspeisen.

Die aus dem Wasserstoff und dem CO₂ im Rauchgas erzeugten Kohlenwasserstoffe lassen sich problemloser als der anfallende Wasserstoff nutzen und z.B. über herkömmliche Strukturen als Kraftstoffe vertreiben.

Als zu produzierende Kraftstoffe in Betracht kommen insbesondere Methanol, aber auch längerkettige Kohlenwasserstoffe.

Aus dem Rauchgas der Kraftwerksanlage wird zweckmäßig ein Teilstrom, ggf. sogar der Gesamtstrom, mit einer Menge an CO₂ abgezweigt, die in einem für die Synthese geeigneten stöchiometrischen Verhältnis zur verfügbaren Wasserstoffmenge steht.

Zweckmäßig wird das Rauchgas vor der Synthese einer Reinigung, z.B. einer Gaswäsche, ggf. Druckgaswäsche, unterzogen.

Zusätzlich können methanhaltige Gase, wie Grubengas, Bio- oder Klärgas im Rahmen der Kohlenwasserstoffsynthese eingesetzt werden.

Bei der Herstellung von Methanol im Rahmen einer z.B. bei etwa 350° C ablaufenden Synthese kann neben dem im Rauchgas vorhandenen Kohlendioxid auch darin enthaltener Sauerstoff an der Synthese beteiligt sein.

Die Erfindung wird nachfolgend anhand von Ausführungsbeispielen und der beiliegenden, sich auf diese Ausführungsbeispiele beziehenden Zeichnungen näher erläutert. Es zeigen:
- Fig. 1: eine schematische Darstellung einer Kraftwerksanlage, in welcher das Verfahren nach der Erfindung durchgeführt wird, und
- Fig. 2: einen Teil einer Kraftwerksanlage gemäß einem zweiten Ausführungsbeispiel für die vorliegende Erfindung.

In einen Kessel 1 einer fossil befeuerten Kraftwerksanlage münden Leitungen 2 bis 4, über welche dem Kessel Verbrennungsluft, Wasser (Deionat) und Brennstoff zugeführt wird.

Durch den Kessel erzeugter Dampf gelangt über eine Leitung 5 in eine Turbine 6, die einen Generator 7 antreibt. Ein Teil der durch den Generator 7 erzeugten Elektroenergie wird, im normalen Betrieb der Kraftwerksanlage, nicht dem Netz sondern einer Elektrolyseeinrichtung 8 zugeführt, die aus Wasser Sauerstoff und Wasserstoff erzeugt. Im Falle einer erforderlichen Stützung der Netzfrequenz durch die Kraftwerksanlage steht der für die Elektrolyse verwendete Elektroenergieanteil kurzfristig zur Einspeisung ins Netz zur Verfügung.

Über eine Leitung 9 aus dem Kessel 1 austretende Rauchgase gelangen über eine Einrichtung 10, welche Stickoxide entfernt, eine Fiftereinrichtung 11 zur Entstaubung und eine Rauchgasentschwefelungsanlage 12 in die Atmosphäre.

Aus dem entschwefelten Rauchgas lässt sich über eine Leitung 13 ein Teilstrom abzweigen und einer Gaswaschanlage 14, hier einer Druckgaswaschanlage, zuführen.

Der Druckgaswäsche unterzogenes Rauchgas gelangt über eine Leitung 16 in einen Synthesereaktor 18, welchem über eine Leitung 17 ferner durch die Elektrolyseeinrichtung 8 erzeugter Wasserstoff zugeführt wird. An den Synthesereaktor 18 schließt sich über eine Leitung 19 eine Trennanlage 20 mit einer Ausgangsleitung 21 an.

In dem Synthesereaktor 18 lässt sich aus dem im Rauchgas enthaltenen Kohlendioxid und dem über die Leitung 17 zugeführten Wasserstoff Methanol synthetisieren, das als Kraftstoff oder Ausgangsprodukt für andere Kraftstoffe verwendet werden kann. Der Methonolsynthese liegen folgende Reaktionsgleichungen zugrunde:

CO₂ + H₂ → CO + H₂O(g)

2H₂ + CO → CH₃OH

CO₂ + 3 H₂ → CH₃OH + H₂O(g).

Auch der im Rauchgas enthaltene Sauerstoffanteil kann gemäß folgender Reaktion an der Methanolsynthese beteiligt sein:

O₂ + 3CO₂ + 11 H₂ → 3CH₃OH + 5H₂O(g).

Im Synthesereaktor 18 gebildetes Methanol und Wasser werden in der nachfolgenden Anlage 20 voneinander getrennt und Methanol wird über die Leitung 21 abgeführt.

Die Energiebilanz des hier bei etwa 350° C ablaufenden Syntheseprozesses ist insgesamt positiv, so dass für die Kraftwerksanlage nutzbare Wärmeenergie zur Verfügung steht.

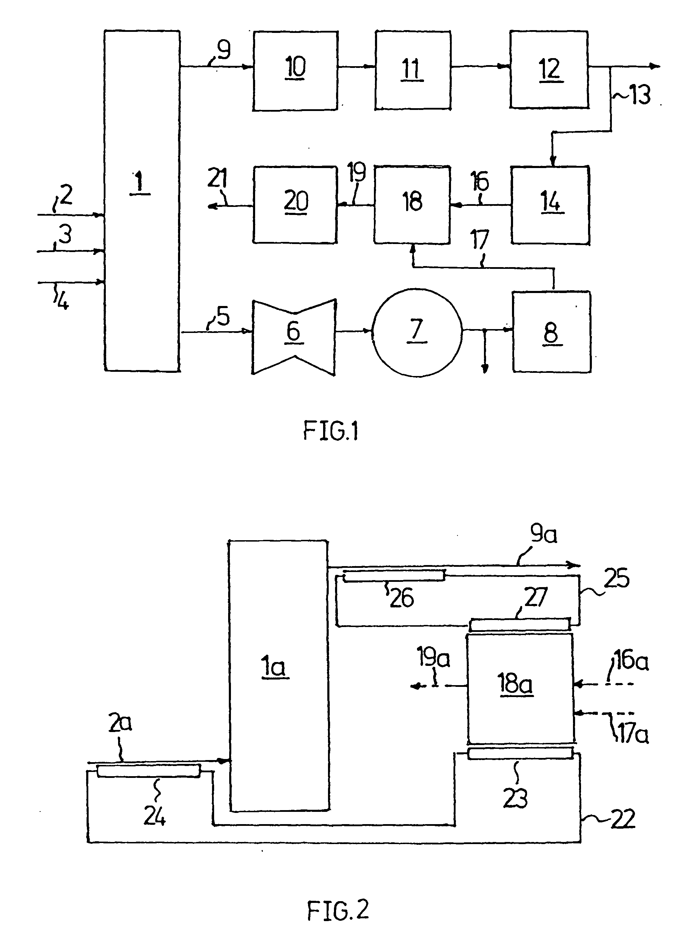

Gemäß dem Ausführungsbeispiel von Fig. 2 lässt sich die, durch einen Synthesereaktor 18a erzeugte Wärmeenergie zur Vorwärmung der einem Kessel 1 a über eine Leitung 2a zugeführten Verbrennungsluft nutzen. Ein hierzu verwendeter Wärmeübertragungskreislauf 22 weist einen Wärme von dem Synthesereaktor 18a abführenden Wärmetauscher 23 und einen Wärme an die Verbrennungsluft abgebenden Wärmetauscher 24 auf.

In Phasen negativer Energiebilanz, z.B. in der Anfahrphase, lässt sich über einen Wärmeübertragungskreislauf 25 mit Wärmetauschern 26 und 27 Wärme aus dem durch eine Ausgangsleitung 9a aus dem Kessel 1 a abgeführten Rauchgas auf den Synthesereaktor 18a übertragen.

Alternativ zu Methanol können auch mehrere -CH₂-Gruppen enthaltende, langerkettige Kohlenwasserstoffe, die insbesondere als Kraftstoffe verwendbar sind, synthetisiert werden, wobei die Synthese auf folgenden Reaktionsgleichungen beruht:

CO₂ + H₂ → CO + H₂O(g)

2H₂ + CO → -(CH₂)- +H₂O(g)

CO₂ + 3H₂ → -(CH₂)- + 2H₂O(g).

## Patentansprüche

1. Verfahren zur Reduzierung der CO₂-Emission fossil befeuerter Kraftwerksanlagen- bei dem das aus Wasserstoff und im Rauchgas der Kraftwerksanlage enthaltendem Kohlendioxid Kohlenwasserstoffe synthetisiert werden und bei dem der Wasserstoff elektrolytisch unter Einsatz eines Teils der durch die Kraftwerksanlage erzeugten Elektroenergie gewonnen wird,
**dadurch gekennzeichnet,**
**dass** zur elektrolytischen Wasserstoffgewinnung eine im Rahmen der Primärregelung der Netzfrequenz kurzfristig abrufbare Reserve der durch die Kraftwerksanlage erzeugten Elektroenergie eingesetzt wird, und dass für die Synthese ggf. erforderliche Wärme aus dem Rauchgas ausgekoppelt und bei der Synthese frei werdende Wärme in die Verbrennungsluft eingekoppelt wird.

2. Verfahren nach Anspruch 1,
**dadurch gekennzeichnet,**
**dass** als Kraftstoff verwendbare Kohlenwasserstoffe, insbesondere flüssige Kohlenwasserstoffe, synthetisiert werden.

3. Verfahren nach Anspruch 1 oder 2,
**dadurch gekennzeichnet,**
**dass** Methanol (CH₃OH) oder/und längerkettige Kohlenwasserstoffe synthetisiert werden.

4. Verfahren nach einem der Ansprüche 1 bis 3,
**dadurch gekennzeichnet,**
**dass** aus dem Rauchgas der Kraftwerksanlage ein Rauchgasstrom mit einer CO₂-Menge abgezweigt wird, die in einem geeigneten stöchiometrischen Verhältnis zur verfügbaren Wasserstoffgasmenge steht.

5. Verfahren nach einem der Ansprüche 1 bis 4,
**dadurch gekennzeichnet,**
**dass** das Rauchgas vor der Synthese einer Reinigung, insbesondere einer Gaswäsche, unterzogen wird.

6. Verfahren nach einem der Ansprüche 1 bis 5,
**dadurch gekennzeichnet,**
**dass** an der Synthese ferner im Rauchgas vorhandener Sauerstoff beteiligt ist.

7. Verfahren nach einem der Ansprüche 1 bis 6,
**dadurch gekennzeichnet,**
**dass** zur Synthese der Kohlenwasserstoffe ferner methanhaltige Gase, wie Grubengas, Bio- oder Klärgas, eingesetzt werden.

8. Fossil befeuerte Kraftwerksanlage, mit einem mit dem Rauchgasableitungssystem (9-12) der Kraftwerksanlage verbundenen Reaktor (18) zur Erzeugung von Kohlenwasserstoffen aus Wasserstoff und im Rauchgas enthaltenem Kohlendioxid sowie mit Einrichtungen (8) zur elektrolytischen Gewinnung des erforderlichen Wasserstoffs unter Einsatz eines Teils der durch die Kraftwerksanlage erzeugten Elektroenergie,
**dadurch gekennzeichnet,**
**dass** die Einrichtungen (8) zur elektrolytischen Wasserstoffgewinnung zur Nutzung einer im Rahmen der Primärregelung der Netzfrequenz kurzfristig abrufbaren Elektroenergiereserve vorgesehen sind, und dass Einrichtungen (25-27) zur Gewinnung für den Synthesereaktor (18a) benötigter Prozesswärme aus dem Rauchgas sowie Einrichtungen (22-24) zur Vorwärmung der Verbrennungsluft für den Kessel (1a) der Krattwerksanlage durch Wärme von dem Synthesereaktor (18a) vorhanden sind.

## Claims

1. Method of reducing CO₂ emission from fossil fuel-fired power stations, in which hydrocarbons are synthesized from hydrogen and carbon dioxide present in the flue gas from the power station and in which the hydrogen is obtained electrolytically using a part of the electric energy generated by the power station,
**characterized**
**in that** a reserve of the electric energy generated by the power station, which can be tapped at short notice for primary regulation of the grid frequency, is used for the electrolytic production of hydrogen and in that the heat which may be required for the synthesis is taken from the flue gas and heat liberated in the synthesis is introduced into the combustion air.

2. Method according to Claim 1, **characterized in that** hydrocarbons which can be used as fuel, in particular liquid hydrocarbons, are synthesized.

3. Method according to Claim 1 or 2, **characterized in that** methanol (CH₃OH) and/or relatively long-chain hydrocarbons are synthesized.

4. Method according to any of Claims 1 to 3, **characterized in that** a flue gas stream containing an amount of CO₂ which is in a suitable stoichiometric ratio to the amount of hydrogen gas available is branched off from the flue gas from the power station.

5. Method according to any of Claims 1 to 4, **characterized in that** the flue gas is subjected to purification, in particular a gas scrub, before the synthesis.

6. Method according to any of Claims 1 to 5, **characterized in that** oxygen present in the flue gas also participates in the synthesis.

7. Method according to any of Claims 1 to 6, **characterized in that** methane-containing gases, for example pit gas, biogas or sewage works gas, are also used for the synthesis of the hydrocarbons.

8. Fossil fuel-fired power station having a reactor (18) for producing hydrocarbons from hydrogen and carbon dioxide present in the flue gas, which reactor is connected to the flue gas discharge system (9-12) of the power station, and having facilities (8) for the electrolytic production of the necessary hydrogen using a part of the electric energy generated by the power station, **characterized**
**in that** the facilities (8) for electrolytic production of hydrogen are provided for utilizing an electric energy reserve, which can be tapped at short notice for primary regulation of the grid frequency, and in that facilities (25-27) for obtaining process heat required for the synthesis reactor (18a) from the flue gas and also facilities (22-24) for preheating the combustion air for the boiler (1a) of the power station by means of heat from the synthesis reactor (18a) are provided.

## Revendications

1. Procédé pour la réduction de l'émission de CO₂ de centrales à combustibles fossiles dans lequel on synthétise à partir d'hydrogène et du dioxyde de carbone contenu dans les gaz de fumée de la centrale à combustible des hydrocarbures et dans lequel l'hydrogène est obtenu électrolytiquement en utilisant une partie de l'énergie électrique produite par la centrale à combustible, **caractérisé en ce qu'**on utilise, pour la production électrolytique d'hydrogène, une réserve, à laquelle on peut faire appel à court terme dans le cadre de la régulation primaire de la fréquence de réseau, de l'énergie électrique produite par la centrale à combustible et **en ce que** la chaleur le cas échéant nécessaire pour la synthèse est découplée des gaz de fumée et couplée à la chaleur libérée lors de la synthèse dans l'air de combustion.

2. Procédé selon la revendication 1, **caractérisé en ce qu'**on synthétise des hydrocarbures utilisables comme carburants, en particulier des hydrocarbures liquides.

3. Procédé selon la revendication 1 ou 2, **caractérisé en ce qu'**on synthétique du méthanol (CH₃OH) et/ou des hydrocarbures à chaîne plus longue.

4. Procédé selon l'une quelconque des revendications 1 à 3, **caractérisé en ce qu'**on dévie des gaz de fumée de la centrale à combustible un flux de gaz de fumée contenant une quantité de CO₂ qui se trouve dans un rapport stoechiométrique approprié par rapport à la quantité d'hydrogène gazeux disponible.

5. Procédé selon l'une quelconque des revendications 1 à 4, **caractérisé en ce que** les gaz de fumée sont soumis, avant la synthèse, à une purification, en particulier un lavage de gaz.

6. Procédé selon l'une quelconque des revendications 1 à 5, **caractérisé en ce que** l'oxygène présent dans les gaz de fumée participe en outre à la synthèse.

7. Procédé selon l'une quelconque des revendications 1 à 6, **caractérisé en ce qu'**on utilise, pour la synthèse des hydrocarbures, en outre des gaz contenant du méthane, tels que le gaz de mine, le biogaz ou le gaz d'égout.

8. Centrale à combustibles fossiles, présentant un réacteur (18) relié à un système d'évacuation des gaz de fumée (9-12) de la centrale à combustible pour la production d'hydrocarbures à partir d'hydrogène et de dioxyde de carbone contenu dans les gaz de fumée, ainsi que des dispositifs (8) pour la production électrolytique de l'hydrogène nécessaire en utilisant une partie de l'énergie électrique produite par la centrale à combustibles, **caractérisée en ce que** les dispositifs (8) pour la production électrolytique d'hydrogène sont prévus, pour l'exploitation d'une réserve d'énergie électrique, à laquelle on peut faire appel à court terme dans le cadre de la régulation primaire de la fréquence de réseau et **en ce que** des dispositifs (25-27) existent pour la production de la chaleur de procédé nécessaire pour le réacteur de synthèse (18a) à partir des gaz de fumée, ainsi que des dispositifs (22-24) pour le préchauffage de l'air de combustion pour la chaudière (1a) de la centrale à combustibles par la chaleur du réacteur de synthèse (18a).
